# EUROPÄISCHE PATENTSCHRIFT

(11) **EP 1 875 936 B1**
(45) Veröffentlichungstag und Bekanntmachung des Hinweises auf die Patenterteilung: **18.11.2009**
(21) Anmeldenummer: 07011119.0
(22) Anmeldetag: 06.06.2007
(51) Int. Cl.: A61M 11/00, A61M 15/00, B05B 17/06

(54) **Verfahren und Vorrichtung ausgelegt für die Reinigung der Verneblermembran in einem Inhalationstherapiegerät**
Method and device configured for cleaning the atomiser membrane in a therapeutic inhalation device
Procédé et dispositif adapté pour le nettoyage de la membrane de vaporisateur dans un appareil de thérapie par inhalation

(30) Priorität: 04.07.2006 DE 102006030833
(43) Veröffentlichungstag der Anmeldung: 09.01.2008
(73) Patentinhaber: PARI Pharma GmbH, 82319 Starnberg (DE)
(72) Erfinder: Gallem, Thomas, 81371 München (DE); Hetzer, Uwe, 81369 München (DE); Holzmann, Philipp, 81667 München (DE)
(74) Vertreter: HOFFMANN EITLE

(56) Entgegenhaltungen:
- US-A1- 2002 023 639
- US-A1- 2004 195 354

## Beschreibung

Die Erfindung betrifft ein Verfahren und eine Reinigungsvorrichtung ausgelegt für die Reinigung der Membran eines Membranaerosolgenerators eines Inhalationstherapiegeräts.

Mit Inhalationstherapiegeräten werden therapeutisch wirksame oder medikamenthaltige Flüssigkeiten mit Hilfe eines Aerosolerzeugers zu einem Aerosol vernebelt, das aus lungengängigen Partikeln besteht. Das erzeugte Aerosol wird einem Patienten im Rahmen einer Inhalationstherapie zum Einatmen dargeboten, wodurch die therapeutisch wirksame Flüssigkeit oder das Medikament in die Atemwege des Patienten gelangen.

Bei besonders effektiven Inhalationstherapiegeräten wird als Aerosolerzeuger ein Membranaerosolgenerator für die Erzeugung des Aerosols eingesetzt. Die zu vernebelnde Flüssigkeit wird in einem Vorratsbehälter bevorratet und der Membran des Membranaerosolgenerators auf der einen Seite, der Flüssigkeitsseite, zugeführt. Wird die Membran des Membranaerosolgenerators in Schwingungen versetzt, wird die Flüssigkeit durch die Öffnungen der Membran hindurch transportiert und auf der anderen Seite der Membran, der Aerosolseite, in Form eines Aerosols abgegeben. Um die Membran in Schwingungen zu versetzen, umfasst der Membranaerosolgenerator neben der Membran einen Schwingungserzeuger, beispielsweise ein ringförmiges piezo-elektrisches Element und ein ringförmiges Substrat, die miteinander und mit der Membran derart verbunden sind, dass ein dem piezo-elektrischen Element zugeführtes Ansteuerungssignal Schwingungen des Schwingungserzeugers veranlasst, die die Schwingungen der Membran hervorrufen.

Als Beispiel eines derartigen Inhalationstherapiegeräts beschreibt EP 1 304 131 eine Inhalationstherapievorrichtung mit einer schwingungsfähigen Membran für die Verneblung einer Flüssigkeit mit einer Schwingungserzeugungseinrichtung, die zumindest eine Anschlusseinrichtung für die Zuführung eines Ansteuersignals aufweist, durch das die Membran in Schwingungen versetzt wird, so dass eine auf einer Seite der Membran anstehende Flüssigkeit durch die Membran hindurch vernebelt wird und auf der anderen Seite der Membran als Aerosol vorliegt.

Inhalationstherapiegeräte mit Membranaerosolgenerator zeichnen sich durch eine hohe Dosisgenauigkeit und kurze Therapiezeiten aus. Insbesondere aufgrund der hervorragenden Steuerbarkeit der Aerosolerzeugung sind qualitativ hochwertige Aerosole erzeugbar, so dass sich Inhalationstherapiegeräte mit Membranaerosolgenerator in besonderem Maße für die Applikation sehr teurer und/oder hochwirksamer Medikamente eignen. Daneben sind Inhalationstherapiegeräte mit Membranaerosolgenerator über einen langen Zeitraum mit zufriedenstellenden Ergebnissen einsetzbar. Es kann nach zahlreichen Therapiesitzungen zu einer Verlangsamung der Aerosolerzeugung kommen, was aber nicht zu einer Verschlechterung der Aerosolqualität führt.

In praktisch allen Anwendungsfällen ist eine übermäßige Verlängerung der Therapiesitzungen nicht hinnehmbar, da stets eine zügige Verabreichung einer bestimmten Dosis des Medikaments angestrebt wird. Denn keinem Patienten/Anwender des Inhalationstherapiegeräts sollen unnötige Therapiezeiten zugemutet werden. Grundsätzlich sind demnach lange Therapiezeiten unerwünscht. Daneben sind die vergleichsweise hohen Herstellungskosten von hochwertigen Inhalationstherapiegeräten mit Membranaerosolgenerator zu beachten. Deshalb besteht das Bedürfnis, die Lebensdauer des Inhalationstherapiegeräts, also den Zeitraum weiter zu verlängern, über den ein Inhalationstherapiegerät mit Membranaerosolgenerator verwendet werden kann, ohne dass es zu einer deutlichen Verschlechterung der therapierelevanten Eigenschaften kommt.

US 2002/0023639 beschreibt einen tragbaren Inhalator für therapeutische Anwendungen, der eine schwingfähige Membran für die Erzeugung eines Aerosols umfasst. Der Membran wird eine zu inhalierende pulverförmige Substanz zugeführt, nachdem sie durch Zuführen einer in einem Behälter bevorrateten Flüssigkeit gelöst wurde. In US2002/0023639 wird beschrieben, dass die Membran des Inhalators gereinigt wird, indem aus dem Flüssigkeitsbehälter, in dem die Flüssigkeit für die Lösung des pulverförmigen Medikaments bevorratet wird, etwas Flüssigkeit der Membran zugeführt und die Membran in Schwingungen versetzt wird. Die Zuführung der Flüssigkeit erfolgt auf der selben Seite, auf der die Flüssigkeit auch für die Erzeugung des Aerosols zugeführt wird.

Vor diesem Hintergrund besteht das von der Erfindung zu lösende Problem darin, einen Weg für eine wirksamere Reinigung der Membran eines Inhalationstherapiegeräts aufzuzeigen, um dadurch den Zeitraum zu verlängern, über den ein Inhalationstherapiegerät mit Membranaerosolgenerator auf hohem Qualitätsniveau verwendet werden kann.

Gelöst wird dieses Problem mit Hilfe eines Verfahrens gemäß Anspruch 1 und einer Reinigungsvorrichtung gemäß Anspruch 4. Vorteilhafte Ausgestaltungen sind jeweils in den Unteransprüchen angegeben.

Die erfindungsgemäße Lösung geht zurück auf Untersuchungen von in Membranaerosolgeneratoren eingesetzten Membranen mit einem Rasterelektronenmikroskop. Diese Untersuchungen haben ergeben, dass trotz regelmäßiger und gründlicher Reinigung des Membranaerosolgenerators gelegentlich ein zunehmend großer Anteil der Löcher durch Material verschiedener Herkunft verstopft wird, wodurch die Geschwindigkeit der Aerosolerzeugung verringert wird, was sich negativ auf die Therapiesitzungsdauer auswirkt.

Die bisher vorgenommene Reinigung der Membran des Membranaerosolgenerators verhindert bzw. beseitigt den nun beobachteten Verschluss einzelner Öffnungen der Membran nicht in dem Maße, wie die Reinigung gemäß der Erfindung, auch wenn in den bekannten Verfahren ausgewählte Reinigungsmittel, beispielsweise Lösungsmittel, eingesetzt werden. Zwar sind die bekannten Arten der Reinigung effektiv, aber nicht in dem nun durch die Erfindung erzielten Umfang.

Erst durch die Zuführung der Reinigungsflüssigkeit auf der Aerosolseite der Membran und die Aktivierung von Membranschwingungen, die die Reinigungsflüssigkeit durch die Membran transportieren, so dass die Reinigungsflüssigkeit auf der Flüssigkeitsseite der Membran abtropft, werden die Verstopfungen entfernt. Mit anderen Worten, eine effektive Reinigung der Membran wird durch einen bislang unbekannten Rückspülbetrieb der Membran erreicht, in dem eine Reinigungsflüssigkeit die Membran in im Vergleich zur Aerosolerzeugung entgegengesetzter Richtung durchläuft. Dabei wird die Membran des Membranaerosolgenerators zu Schwingungen angeregt und übernimmt somit eine aktive Rolle beim Reinigungsprozess im Gegensatz zu bisher bekannten Reinigungsprozessen, in denen die Reinigungsflüssigkeit bzw. die Art der Zuführung der Reinigungsflüssigkeit eine aktive Rolle übernimmt.

Im Folgenden wird die vorliegende Erfindung unter Bezugnahme auf die Zeichnungen genauer erläutert, in denen zeigt:
- Fig. 1: ein erstes Ausführungsbeispiel einer Reinigungsvorrichtung ausgelegt für die Reinigung einer schwingungsfähigen Membran eines Inhalationstherapiegeräts gemäß der Erfindung;
- Fig. 2: ein zweites Ausführungsbeispiel der erfindungsgemäßen Reinigungsvorrichtung eines Inhalationstherapiegeräts gemäß der Erfindung;
- Fig. 3: ein drittes Ausführungsbeispiel einer erfindungsgemäßen Reinigungsvorrichtung eines Inhalationstherapiegeräts gemäß der Erfindung;
- Fig. 4A: ein Inhalationstherapiegerät mit einem Membranaerosolgenerator;
- Fig. 4B: ein viertes Ausführungsbeispiel der erfindungsgemäßen Reinigungsvorrichtung eines Inhalationstherapiegeräts gemäß der Erfindung.

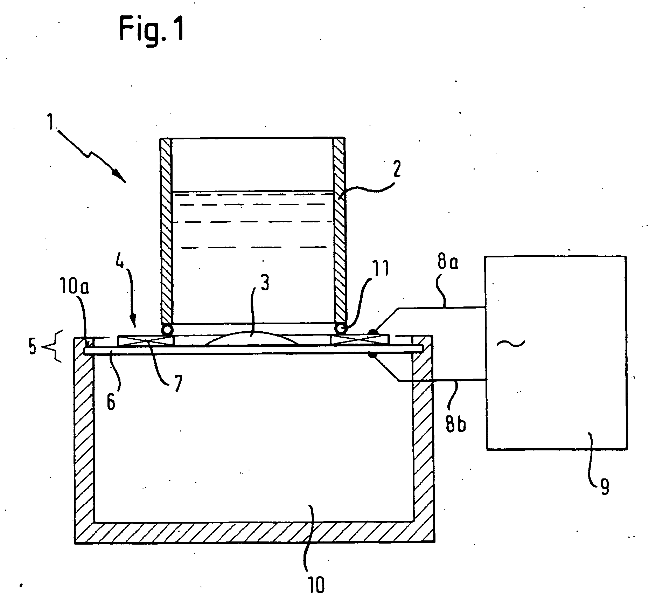

Fig. 1 stellt ein erstes vorteilhaftes Ausführungsbeispiel einer erfindungsgemäßen Reinigungsvorrichtung ausgelegt für die Reinigung einer schwingungsfähigen Membran eines Membranaerosolgenerators eines Inhalationstherapiegerätes dar. Die gezeigte Reinigungsvorrichtung 1 umfasst eine Flüssigkeitszuführungseinrichtung 2 ausgelegt für die Zuführung einer Reinigungsflüssigkeit zur Aerosolseite einer Membran 3. Die Zuführung der Reinigungsflüssigkeit erfolgt erfindungsgemäß auf der Seite der Membran 3, auf der sonst während des Vernebelns einer Flüssigkeit das von dem Membranaerosolgenerator erzeugte Aerosol abgegeben wird.

Der Membranaerosolgenerator 4 umfasst neben der schwingungsfähigen Membran 3 einen Schwingungserzeuger 5, der in dem gezeigten Ausführungsbeispiel aus einem ringförmigen Substrat 6 und einem damit verbundenen ringförmigen piezo-elektrischen Element 7 besteht. Bei dem gezeigten Ausführungsbeispiel besitzt der Schwingungserzeuger 5 damit eine rotationssymmetrische Grundstruktur, deren Rotationsachse in der Zeichnungsebene der Fig. 1 liegt.

Wie Fig. 1 ferner zeigt, sind Anschlussleitungen 8a, 8b einerseits mit dem Schwingungserzeuger 5 und andererseits erfindungsgemäß mit einer Schwingungsaktivierungseinrichtung 9 für die Aktivierung von Membranschwingungen verbunden.

Gemäß dem in Fig. 1 gezeigten Ausführungsbeispiel führt die Schwingungsaktivierungseinrichtung 9 dem Schwingungserzeuger 5 des Membranaerosolgenerators 4 ein elektrisches Ansteuerungssignal zu. Wenn das Ansteuerungssignal zugeführt wird, wird die schwingungsfähige Membran 3 durch den Schwingungserzeuger 5 in Schwingungen versetzt und die Reinigungsflüssigkeit erfindungsgemäß von der Aerosolseite der Membran 3 durch die Membran 3 zur Flüssigkeitsseite der Membran 3 gefördert.

Vorteilhafterweise wird, wie Fig. 1 zeigt, die durch die Öffnungen der Membran geförderte Reinigungsflüssigkeit in einem Auffangbehälter 10 aufgefangen, an dem der Membranaerosolgenerator 4 geeignet angeordnet bzw. angebracht ist. Beispielsweise ist gemäß Fig. 1 das ringförmige Substrat 6 an seinem äußeren Umfang in einer Nut 10a des Auffangbehälters 10 fixiert.

Bei dem in Fig. 1 gezeigten Ausführungsbeispiel ist die Flüssigkeitszuführungseinrichtung 2 in Form eines an beiden Stirnseiten offenen Hohlzylinders realisiert. Die eine Stirnseite des Hohlzylinders 2 ist so positioniert, dass diese Stirnseite von der Membran 3 bzw. dem Membranaerosolgenerator 4 dicht abgeschlossen wird. An der der Membran zugewandten Stirnseite ist dazu vorteilhaft eine ringförmige Dichtung 11 angeordnet, die an der Membran 3 bzw. dem Membranaerosolgenerator 4 derart anliegt, dass eine in den Hohlzylinder eingefüllte Reinigungsflüssigkeit nicht an der der Membran zugewandten Stirnseite des Hohlzylinders austritt, sondern an der Membran 3 ansteht. Sofern der Hohlzylinder ein ausreichendes Eigengewicht aufweist, um die für die Zuführung der Reinigungsflüssigkeit zu erzielende Dichtigkeit (mit oder ohne Dichtung 11) zu erreichen, sind weitere Maßnahmen zur Fixierung der Flüssigkeitszuführungseinrichtung 2 nicht erforderlich. Andernfalls (oder zusätzlich) können aber Fixierungen für die Flüssigkeitszuführungseinrichtung 2 vorgesehen werden, die die Flüssigkeitszuführungseinrichtung 2 zuverlässig an der für die Zuführung der Reinigungsflüssigkeit geeigneten Position halten.

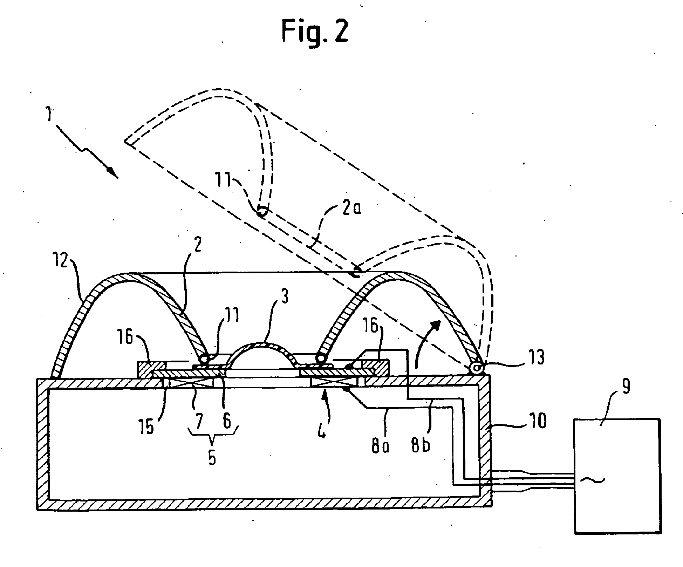

Fig. 2 stellt eine zweite vorteilhafte Ausführungsform einer erfindungsgemäßen Reinigungsvorrichtung dar. Bei der in Fig. 2 gezeigten Reinigungsvorrichtung 1 ist die Flüssigkeitszuführungseinrichtung 2 für die Zuführung einer Reinigungsflüssigkeit zur Aerosolseite der Membran 3 trichterförmig ausgestaltet. Ferner ist die trichterförmige Flüssigkeitszuführungseinrichtung 2 einstückig mit einem Deckel 12 ausgebildet, der um eine Achse 13 schwenkbar an dem Auffangbehälter 10 befestigt ist. Im geschlossenen Zustand ist die trichterförmige Flüssigkeitszuführungseinrichtung 2 so positioniert, dass das freie offene Ende 2a an der Membran 3 oder dem Membranaerosolgenerator 4 anliegt, so dass eine in die trichterförmige Flüssigkeitszuführungseinrichtung 2 eingefüllte Reinigungsflüssigkeit an der Membran 3 ansteht. Vorteilhafterweise ist an dem freien offenen Ende der Flüssigkeitszuführungseinrichtung 2 eine Dichtung 11 vorgesehen, um ein unerwünschtes Austreten von Reinigungsflüssigkeit aus dem durch die Flüssigkeitszuführungseinrichtung 2 und die Membran 3 begrenzten Volumen zu verhindern.

Für das Entnehmen und das Einsetzen des Membranaerosolgenerators 4 in die Reinigungsvorrichtung 1 gemäß dem zweiten Ausführungsbeispiel kann die Flüssigkeitszuführungseinrichtung 2 zusammen mit dem Deckel 12 um die Achse 13 geschwenkt werden. Der Membranaerosolgenerator 4 ist an einer an dem Auffangbehälter vorgesehenen Aufnahme 15, beispielsweise einer angepassten Öffnung in der dem Deckel zugewandten Oberfläche des Auffangbehälters 10, durch eine oder mehrere Halteeinrichtungen 16, die beispielsweise den Membranaerosolgenerator 4 einklemmen, lösbar fixiert.

Wie in der ersten Ausführungsform erfolgt auch in der zweiten Ausführungsform die Zuführung der Reinigungsflüssigkeit erfindungsgemäß auf der Aerosolseite der Membran 3, also auf der Seite, auf der sonst während des Vernebelns einer Flüssigkeit das von dem Membranaerosolgenerator erzeugte Aerosol abgegeben wird. Wird der Membranaerosolgenerator 4 angesteuert, so dass die Membran 3 in Schwingungen versetzt wird, tritt die Reinigungsflüssigkeit auf der anderen Seite der Membran 3 aus, also auf der Seite, auf der sonst während eines Verneblerbetriebs die zu vernebelnde Flüssigkeit ansteht.

Dem Membranaerosolgenerator 4 wird, wie beim ersten Ausführungsbeispiel, ein Ansteuersignal über Anschlussleitungen 8a und 8b von einer Schwingungsaktivierungseinrichtung 9 zugeführt.

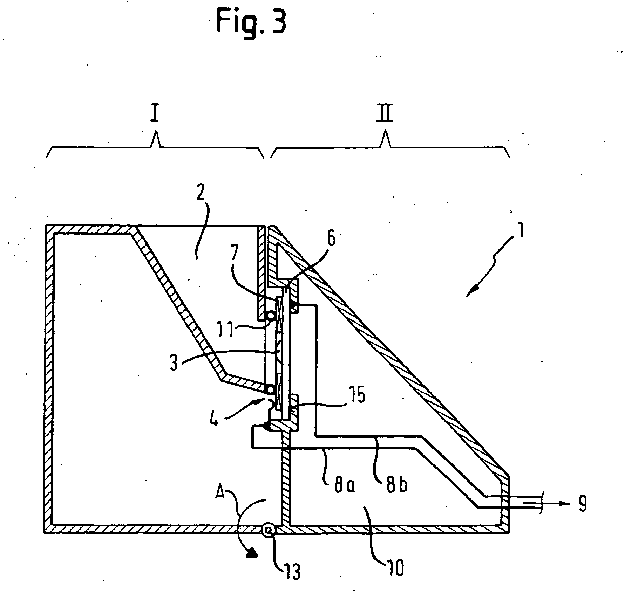

Figur 3 zeigt ein drittes Ausführungsbeispiel einer erfindungsgemäßen Reinigungsvorrichtung 1. Bei diesem Ausführungsbeispiel ist der Membranaerosolgenerator 4 nicht horizontal, sondern vertikal in der Reinigungsvorrichtung angeordnet, um zu veranschaulichen, dass das erfindungsgemäße Verfahren auch durchführbar ist, wenn die Membran 3 des Membranaerosolgenerators nicht horizontal angeordnet ist. Letztlich kann die Membran 3 des Membranaerosolgenerators 4 beliebig angeordnet werden, so lange mit Hilfe der Flüssigkeitszuführungseinrichtung 2 die Reinigungsflüssigkeit der Aerosolseite der Membran 3 des Membranaerosolgenerators 4 zugeführt und die Membran 3 derart in Schwingungen versetzt wird, dass die Reinigungsflüssigkeit von der Aerosolseite auf die Flüssigkeitsseite der Membran 3 gefördert wird.

Bei dem in Figur 3 gezeigten dritten Ausführungsbeispiel ist die Flüssigkeitszuführungseinrichtung 2 ein teilweise abgeschrägter Behälter, der eine vertikale Öffnung aufweist, die so angeordnet ist, dass die Reinigungsflüssigkeit an der Aerosolseite der Membran 3 ansteht. An der Öffnung der Flüssigkeitszuführungseinrichtung 2 ist vorzugsweise eine Dichtung 11 vorgesehen, die ein unerwünschtes Austreten der Reinigungsflüssigkeit an der Kontaktstelle zwischen Trichter/Dichtung und Membran/Membranaerosolgenerator verhindert.

Wie der in Figur 3 dargestellte Pfeil A andeutet, kann der Teil I der Reinigungsvorrichtung 1, in dem die Flüssigkeitszuführungseinrichtung 2 ausgebildet ist, um eine Drehachse geschwenkt werden, wodurch die Aufnahme 15 für den Membranaerosolgenerator 4 freigegeben wird, die in einem anderen Teil II der Reinigungsvorrichtung 1 ausgebildet ist. In die Aufnahme 15 wird der Membranaerosolgenerator 4 eingelegt und dabei mit Anschlussleitungen 8a und 8b verbunden, über die ein Ansteuerungssignal zu dem Membranaerosolgenerator 4 herangeführt wird. Wenn der erste Gehäuseteil I der Reinigungsvorrichtung 1 in die in Figur 3 gezeigte Position geschwenkt ist, wird der Membranaerosolgenerator 4 bzw. die Membran 3 in der Aufnahme fixiert, da die Dichtung 11 auf den Membranaerosolgenerator 4 mechanisch einwirkt. Der zweite Gehäuseteil II der Reinigungsvorrichtung 1 gemäß Figur 3 dient als Auffangraum 10 für die durch die Membran hindurch geförderte Reinigungsflüssigkeit.

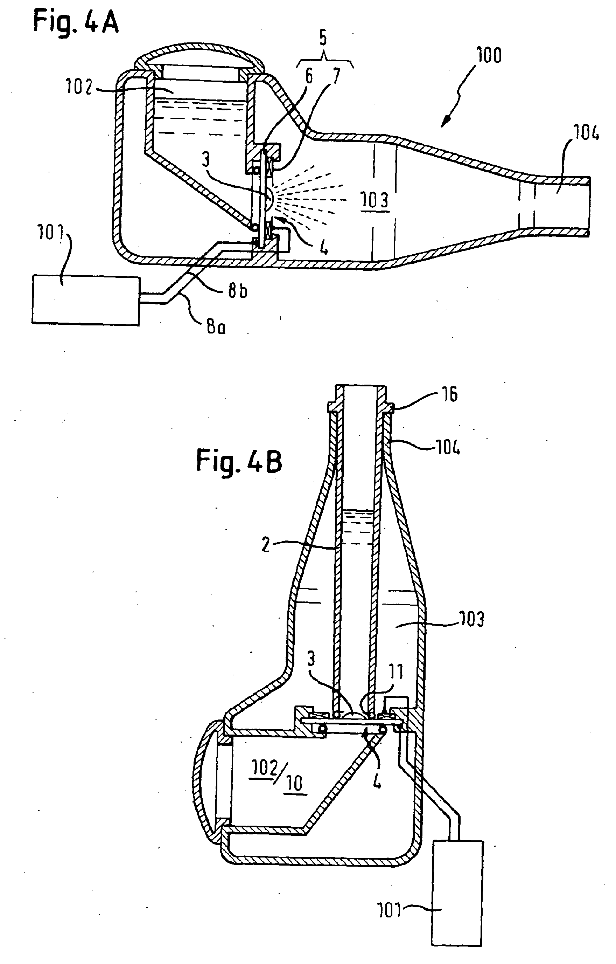

Figur 4A zeigt ein Inhalationstherapiegerät 100 mit einem Membranaerosolgenerator 4, der neben der Membran 3 einen Schwingungserzeuger umfasst, der ein ringförmiges Substrat 6 und ein ringförmiges piezo-elektrisches Element 7 aufweist, die miteinander und mit der Membran 3 derart verbunden sind, dass die Membran 3 in Schwingungen versetzt wird, wenn der Schwingungserzeuger 5 durch ein elektrisches Ansteuersignal zu Schwingungen angeregt wird. Das elektrische Ansteuerungssignal wird über Zuleitungen 8a, 8b zugeführt, die an ein Steuergerät 101 des Inhalationstherapiegeräts 100 angeschlossen sind. In ein Flüssigkeitsreservoir 102 des Inhalationstherapiegeräts wird die zu vernebelnde Flüssigkeit eingefüllt und steht während des Vernebelbetriebs auf der Flüssigkeitsseite der Membran 3 an. Wenn von dem Steuergerät 101 ein Ansteuersignal dem Membranaerosolgenerator 4 zugeführt wird, wird die Membran 3 in Schwingungen versetzt, so dass die zu vernebelnde Flüssigkeit von der Flüssigkeitsseite auf die Aerosolseite der Membran 3 transportiert wird. Das Aerosol wird von dem Membranaerosolgenerator 4 in eine Mischkammer 103 abgegeben, aus der ein Patient das Aerosol über ein Mundstück 104 einatmet. Obwohl in Fig. 4A einstückig dargestellt, ist das Mundstück 104 in vielen Fällen von der Mischkammer 103 abnehmbar.

Bei einem derart ausgestalteten Inhalationstherapiegerät umfasst, wie Figur 4B zeigt, die Reinigungsvorrichtung gemäß der Erfindung eine Flüssigkeitszuführungseinrichtung 2, die in Form eines Rohres ausgebildet ist, das in das Mundstück 104 des Inhalationstherapiegeräts eingeführt wird. Die Länge der Flüssigkeitszuführungseinrichtung 2 richtet sich nach der Größe des Mundstücks 104 und der Mischkammer 103 und ist so groß ausgelegt, dass eine offene Stirnseite der Flüssigkeitszuführungseinrichtung 2 die Membran 3 erreicht, so dass die an dieser Stirnseite vorgesehene Dichtung 11 an der Membran 3 oder dem Membranaerosolgenerator 4 anliegt. Sofern das Mundstück 104 von der Mischkammer 103 abnehmbar ist, kann die rohrartige Flüssigkeitszuführungseinrichtung 2 in ihren Abmessungen auch an die Mischkammer 103 angepasst werden.

Vorzugsweise besitzt die Flüssigkeitszuführungseinrichtung 2 einen Anschlag 16, der an dem Öffnungsrand des Mundstücks 104 bzw. der Mischkammer 103 des Inhalationstherapiegeräts anschlägt, und dadurch verhindert, dass die Flüssigkeitszuführungseinrichtung 2 zu weit in das Inhalationstherapiegerät 100 eingeführt wird und dabei die Membran 3 oder den Membranaerosolgenerator 4 beschädigt. Daneben läuft das Rohr 2 vorzugsweise konisch zu, um das Einführen in das Mundstück bzw. die Mischkammer zu erleichtern und um an dem einem Ende einen stirnseitigen Querschnitt, der auf die Membran 3 bzw. den Membranaerosolgenerator 4 angepasst ist, an dem entgegengesetzten Ende einen stirnseitigen Querschnitt zu erreichen, der an die Öffnung des Mundstücks 104 bzw. der Mischkammer 103 angepasst ist.

In das Rohr 2, das bei dem vierten Ausführungsbeispiel die Flüssigkeitszuführungseinrichtung der erfindungsgemäßen Reinigungsvorrichtung bildet, wird die Reinigungsflüssigkeit eingefüllt, so dass diese auf der Aerosolseite der Membran 3 während des Reinigungsvorgangs ansteht. Die Membran 3 wird mit Hilfe des Steuergeräts 101 in Schwingungen versetzt, das auch zur Ansteuerung des Membranaerosolgenerators 4 für die Erzeugung eines Aerosols verwendet wird. Dadurch wird auf vorteilhafte Weise das Steuergerät 101 des Inhalationstherapiegeräts 100 auch für die Reinigung verwendet. Wenn ein Ansteuersignal von dem Steuergerät 101 zu dem Membranaerosolgenerator 4 zugeführt wird, wird die Membran 3 in Schwingungen versetzt, so dass die Reinigungsflüssigkeit, die mit Hilfe der Flüssigkeitszuführungseinrichtung 2 der Aerosolseite der Membran 3 zugeführt wird, durch die Membran 3 transportiert wird und im Flüssigkeitsreservoir 102 aufgefangen wird. Das Flüssigkeitsreservoir 102 des Inhalationstherapiegeräts 100 dient dabei als Auffangbehälter 10 für die Reinigungsflüssigkeit, die durch die Membran 3 hindurchgefördert wurde. Um zu vermeiden, dass versehentlich die im Flüssigkeitsreservoir 102 aufgefangene Reinigungsflüssigkeit vernebelt und eingeatmet wird, sollte aber vorzugsweise bei der Reinigung der Deckel des Flüssigkeitsreservoirs 102 entfernt werden. Um die dann aus dem Flüssigkeitsreservoir austretende Reinigungsflüssigkeit aufzufangen, muss ein separater Auffangbehälter 10 vorgesehen werden. Auf diesem Weg wird auch bei dem in Fig. 4B gezeigten Ausführungsbeispiel der allen Ausbildungen gemäß der Erfindung innewohnende weitere Vorteil sicher erreicht, dass keine Reinigungsflüssigkeit versehentlich vernebelt wird.

Bei dem zuletzt beschriebenen vierten Ausführungsbeispiel einer erfindungsgemäßen Reinigungsvorrichtung ist von besonderem Vorteil, dass die Reinigungsflüssigkeit der Aerosolseite der Membran zugeführt wird, da auf diesem Weg verhindert wird, dass eine Reinigungsflüssigkeit, die in das Flüssigkeitsreservoir eingefüllt ist, versehentlich vernebelt und eingeatmet wird. Die Zuführung der Reinigungsflüssigkeit auf der Aerosolseite der Membran verhindert, dass das Inhalationstherapiegerät möglicherweise zum Vernebeln einer Flüssigkeit verwendet wird.

Darüber hinaus ist das vierte Ausführungsbeispiel vorteilhaft, weil das Steuergerät 101 des Inhalationstherapiegeräts 100 als erfindungsgemäße Schwingungsaktivierungseinrichtung 9 verwendet wird, um die Membran 3 in Schwingungen zu versetzen, damit die Reinigungsflüssigkeit von der Aerosolseite auf die Flüssigkeitsseite transportiert wird. Auch bei den zuvor beschriebenen Ausführungsbeispielen kann das Steuergerät eines Inhalationstherapiegeräts verwendet werden, um das Ansteuersignal dem Membranaerosolgenerator zuzuführen, damit die Membran in Schwingungen versetzt wird, um den gewünschten Transport der Reinigungsflüssigkeit von der Aerosolseite auf die Flüssigkeitsseite der Membran zu bewerkstelligen. Jedoch kann bei einer erfindungsgemäßen Reinigungsvorrichtung auch ein von dem Inhalationstherapiegerät unabhängiges Ansteuergerät als Schwingungsaktivierungseinrichtung verwendet werden, was die Möglichkeit bietet, andere Ansteuersignale, zum Beispiel in anderen Frequenzbereichen oder mit anderen Wellenformen bereitzustellen. Jedoch hat sich gezeigt, dass bei der Verwendung des Steuergeräts des Inhalationstherapiegeräts bei den Betriebsbedingungen, die auch für die Verneblung der Flüssigkeit zur Erzeugung eines Aerosols verwendet werden, gute Reinigungserfolge erzielt werden.

Mit einer erfindungsgemäßen Reinigungsvorrichtung kann ein hochwirksames Verfahren zur Reinigung einer schwingungsfähigen Membran 3 eines Membranaerosolgenerators 4 eines Inhalationstherapiegeräts durchgeführt werden. Dazu wird erfindungsgemäß der Membran 3 auf der Aerosolseite eine Reinigungsflüssigkeit zugeführt. Das heißt, die Zuführung erfolgt auf der Membranseite, auf der im üblichen Verneblerbetrieb des Membranaerosolgenerators das erzeugte Aerosol austritt. Ferner wird erfindungsgemäß die Membran 3 derart in Schwingungen versetzt, dass die Reinigungsflüssigkeit durch die Öffnungen der Membran 3 auf die Flüssigkeitsseite der Membran gefördert wird.

Als Reinigungsflüssigkeit kann destilliertes Wasser, Ethanol, ein Lösungsmittel oder eine andere geeignete Flüssigkeit eingesetzt werden.

Erfindungsgemäß erfolgt die Aktivierung der Membranschwingungen vorzugsweise bei der Frequenz, die auch für die Verneblung einer Flüssigkeit im üblichen Verneblerbetrieb eingesetzt wird. Jedoch kann die Ansteuerung auch auf andere Weise erfolgen, so dass sich das zugeführte Ansteuersignal während der Reinigung der Membran von dem Ansteuersignal eines Verneblerbetriebs, insbesondere hinsichtlich Amplitude, Frequenz und Frequenzwechsel, Dauer und Sequenz der Aktivierungszeiträume unterscheidet.

## Patentansprüche

1. Verfahren zur Reinigung einer schwingungsfähigen Membran (3) eines Membranaerosolgenerators (4) eines Inhalationstherapiegeräts, **dadurch gekennzeichnet, dass**
der Membran (3) auf der Aerosolseite eine Reinigungsflüssigkeit zugeführt wird, und
die Membran (3) derart in Schwingungen versetzt wird, dass die Reinigungsflüssigkeit durch die Öffnungen der Membran (3) auf die Flüssigkeitsseite der Membran gefördert wird.

2. Verfahren nach Anspruch 1, **dadurch gekennzeichnet, dass** die Reinigungsflüssigkeit destilliertes Wasser, Ethanol, ein Lösungsmittel oder eine Mischung aus diesen oder anderen Reinigungsflüssigkeiten ist.

3. Verfahren nach Anspruch 1 oder 2, **dadurch gekennzeichnet, dass** die Membran (3) auf die Art in Schwingungen versetzt wird, wie im Verneblerbetrieb.

4. Reinigungsvorrichtung ausgelegt für die Reinigung einer schwingungsfähigen Membran eines Membranaerosolgenerators eines Inhalationstherapiegeräts mit
- einer Flüssigkeitszuführungseinrichtung (2) ausgelegt für die Zuführung einer Reinigungsflüssigkeit auf die Aerosolseite der Membran, und
- einer Schwingungsaktivierungseinrichtung (9) ausgelegt für die Aktivierung von Membranschwingungen, so dass die Reinigungsflüssigkeit durch die Öffnungen der Membran (3) auf die Flüssigkeitsseite der Membran (3) gefördert wird.

5. Reinigungsvorrichtung nach Anspruch 4, **dadurch gekennzeichnet, dass** die Flüssigkeitszuführungseinrichtung (2) ein Hohlzylinder ist, der mit einer Stirnseite an der Membran (3) oder dem Membranaerosolgenerator (4) derart angeordnet ist, dass eine in den Hohlzylinder (2) eingefüllte Reinigungsflüssigkeit an der Membran (3) ansteht.

6. Reinigungsvorrichtung nach Anspruch 5, **dadurch gekennzeichnet, dass** der Hohlzylinder als längliches Rohr (2) ausgebildet ist, das für das Einsetzen in ein Mundstück (104) oder in eine Mischkammer (103) eines Inhalationstherapiegeräts (100) ausgelegt ist.

7. Reinigungsvorrichtung nach Anspruch 6, **dadurch gekennzeichnet, dass** das längliche Rohr (2) einen Anschlag (16) aufweist, der das Einführen des Rohres in das Mundstück (104) eines Inhalationstherapiegeräts (100) begrenzt.

8. Reinigungsvorrichtung nach Anspruch 6 oder 7, **dadurch gekennzeichnet, dass** das längliche Rohr (2) zu dem für die Anordnung an der Membran vorgesehenen Ende konisch zuläuft.

9. Reinigungsvorrichtung nach Anspruch 4, **dadurch gekennzeichnet, dass** die Flüssigkeitszuführungseinrichtung (2) trichterförmig ist und mit einer Öffnung an der Membran (3) oder dem Membranaerosolgenerator (4) derart angeordnet ist, dass eine in die trichterförmige Flüssigkeitszuführungseinrichtung (2) eingefüllte Reinigungsflüssigkeit an der Membran (3) ansteht.

10. Reinigungsvorrichtung nach einem der Ansprüche 4 bis 9, **dadurch gekennzeichnet, dass** an der Flüssigkeitszuführungseinrichtung (2) eine Dichtung (11) vorgesehen ist, die ein Austreten der Reinigungsflüssigkeit an der Membran (3) bzw. an dem Membranaerosolgenerator (4) verhindert.

11. Reinigungsvorrichtung nach einem der Ansprüche 4 bis 10, **dadurch gekennzeichnet, dass** ein Auffangbehälter (10) für das Auffangen der auf der Flüssigkeitsseite der Membran austretende Reinigungsflüssigkeit vorgesehen ist.

12. Reinigungsvorrichtung nach Anspruch 10, **dadurch gekennzeichnet, dass** in dem Auffangbehälter (10) eine Aufnahme (15) für die Anordnung des Membranaerosolgenerators (4) vorgesehen ist.

13. Reinigungsvorrichtung nach Anspruch 11, **dadurch gekennzeichnet, dass** die Flüssigkeitszuführeinrichtung (2) und der Auffangbehälter (10) zueinander um eine Achse (13) schwenkbar ausgebildet sind.

14. Reinigungsvorrichtung nach Anspruch 13, **dadurch gekennzeichnet, dass** die Flüssigkeitszuführeinrichtung (2) einstückig mit einem Deckel (12) ausgebildet ist.

15. Reinigungsvorrichtung nach einem der Ansprüche 4 bis 14, **dadurch gekennzeichnet, dass** die Schwingungsaktivierungseinrichtung (9) ein Ansteuergerät ist, das ein vorzugsweise elektrisches Ansteuersignal für die Ansteuerung des Membranaerosolgenerators (4) abgibt.

16. Reinigungsvorrichtung nach Anspruch 15, **dadurch gekennzeichnet, dass** das Ansteuergerät (9) das Steuergerät (101) des Inhalationstherapiegeräts (100) ist.

17. Reinigungsvorrichtung nach Anspruch 15 oder 16, **dadurch gekennzeichnet, dass** das Ansteuersignal der Schwingungsaktivierungseinrichtung (9) dem Ansteuersignal entspricht, das während eines Verneblerbetriebs dem Membranaerosolgenerator (4) zugeführt wird.

18. Reinigungsvorrichtung nach Anspruch 15 oder 16, **dadurch gekennzeichnet, dass** das Ansteuersignal der Schwingungsaktivierungseinrichtung (9) sich von dem Ansteuersignal eines Verneblerbetriebs, insbesondere hinsichtlich Amplitude, Frequenz und Frequenzwechsel, Dauer und Sequenz der Aktivierungszeiträume unterscheidet.

## Claims

1. Method for cleaning an oscillatable membrane (3) of a membrane aerosol generator (4) of an inhalation therapy device, **characterised in that**
the membrane (3) is supplied on the aerosol side with a cleaning liquid , and
the membrane (3) is caused to oscillate in such a way that the cleaning liquid is conveyed through the openings of the membrane (3) on the liquid side of the membrane.

2. Method according to claim 1, **characterised in that** the cleaning liquid is distilled water, ethanol, a solvent or a mixture of these or other cleaning liquids.

3. Method according to claim 1 or 2, **characterised in that** the membrane (3) is caused to oscillate in the same manner as in the nebulising mode.

4. Cleaning device designed for cleaning an oscillatable membrane of a membrane aerosol generator of an inhalation therapy device comprising
- liquid supply means (2) designed for supplying a cleaning liquid to the aerosol side of the membrane, and
- an oscillation activating means (9) designed for activating membrane oscillations in such a way that the cleaning liquid is conveyed through the openings of the membrane (3) to the liquid side of the membrane (3).

5. Cleaning device according to claim 4, **characterised in that** the liquid supply means (2) is a hollow cylinder that is arranged with one front end on the membrane (3) or the membrane aerosol generator (4) in such a way that a cleaning liquid filled into the hollow cylinder (2) is disposed on the membrane (3).

6. Cleaning device according to claim 5, **characterised in that** the hollow cylinder is configured as an elongated tube (2) that is designed for insertion into a mouthpiece (104) or a mixing chamber (103) of an inhalation therapy device (100).

7. Cleaning device according to claim 6, **characterised in that** the elongated tube (2) has a stop (16) that limits insertion of the tube into the mouthpiece (104) of an inhalation therapy device (100).

8. Cleaning device according to claim 6 or 7, **characterised in that** the elongated tube (2) extends in a conical manner towards the end provided for arrangement on the membrane.

9. Cleaning device according to claim 4, **characterised in that** the liquid supply means (2) is funnel-shaped and is arranged with an opening on the membrane (3) or the membrane aerosol generator (4) in such a manner that a cleaning liquid filled into the funnel-shaped liquid supply means (2) is disposed on the membrane (3).

10. Cleaning device according to one of the claims 4 to 9, **characterised in that** a seal (11) is provided on the liquid supply means (2), which prevents the escape of the cleaning liquid at the membrane (3) or the membrane aerosol generator (4).

11. Cleaning device according to one of the claims 4 to 10, **characterised in that** a collecting receptacle (10) is provided for collecting the cleaning liquid discharging on the liquid side of the membrane.

12. Cleaning device according to claim 10, **characterised in that** an accommodating member (15) for the arrangement of the membrane aerosol generator (4) is provided in the collecting receptacle (10).

13. Cleaning device according to claim 11, **characterised in that** the liquid supply means (2) and the collecting receptacle (10) are configured so as to be pivotable about an axis (13) in relation to one another.

14. Cleaning device according to claim 13, **characterised in that** the liquid supply means (2) is configured integrally with a lid (12).

15. Cleaning device according to one of the claims 4 to 14, **characterised in that** the oscillation activating means (9) is a control means that emits a preferably electric control signal to control the membrane aerosol generator (4).

16. Cleaning device according to claim 15, **characterised in that** the control means (9) is the control device (101) of the inhalation therapy device (100).

17. Cleaning device according to claim 15 or 16, **characterised in that** the control signal of the oscillation activating means (9) corresponds to the control signal that is supplied to the membrane aerosol generator (4) in a nubulising mode.

18. Cleaning device according to claim 15 or 16, **characterised in that** the control signal of the oscillation activating means (9) differs from the control signal of a nebulising mode in particular as regards amplitude, frequency and change of frequency, duration and sequence of the activation periods.

## Revendications

1. Procédé pour nettoyer une membrane pouvant osciller (3) d'un générateur d'aérosol à membrane (4) d'un appareil d'inhalation thérapeutique, **caractérisé en ce**
- **qu**'un liquide de nettoyage est amené à la membrane (3) du côté de l'aérosol, et
- que la membrane (3) est mise en oscillation de telle sorte que le liquide de nettoyage passe à travers les ouvertures de la membrane (3) du côté du liquide de la membrane.

2. Procédé selon la revendication 1, **caractérisé en ce que** le liquide de nettoyage est de l'eau distillée, de l'éthanol, un solvant ou un mélange de ceux-ci ou d'autres liquides de nettoyage.

3. Procédé selon la revendication 1 ou 2, **caractérisé en ce que** la membrane (3) est mise en oscillation comme pour un fonctionnement en nébuliseur.

4. Dispositif de nettoyage conçu pour le nettoyage d'une membrane pouvant osciller d'un générateur d'aérosol à membrane d'un appareil d'inhalation thérapeutique, avec
- un dispositif d'amenée de liquide (2) conçu pour amener un liquide de nettoyage du côté aérosol de la membrane, et
- un dispositif d'activation d'oscillation (9) conçu pour l'activation d'oscillations de membrane, de telle sorte que le liquide de nettoyage passe à travers les ouvertures de la membrane (3) du côté du liquide de la membrane (3).

5. Dispositif de nettoyage selon la revendication 4, **caractérisé en ce que** le dispositif d'amenée de liquide (2) est un cylindre creux qui est agencé avec un côté frontal contre la membrane (3) ou contre le générateur d'aérosol à membrane (4), de telle sorte qu'un liquide de nettoyage versé dans le cylindre creux (2) est en contact avec la membrane (3).

6. Dispositif de nettoyage selon la revendication 5, **caractérisé en ce que** le cylindre creux est conçu comme un tube de forme allongée (2) qui est dimensionné pour être introduit dans une embouchure (104) ou dans une chambre de mélange (103) d'un appareil d'inhalation thérapeutique (100).

7. Dispositif de nettoyage selon la revendication 6, **caractérisé en ce que** le tube de forme allongée (2) comporte une butée (16) qui limite l'introduction du tube dans l'embouchure (104) d'un appareil d'inhalation thérapeutique (100).

8. Dispositif de nettoyage selon la revendication 6 ou 7, **caractérisé en ce que** le tube de forme allongée (2) s'étend de façon conique vers l'extrémité prévue pour l'agencement contre la membrane.

9. Dispositif de nettoyage selon la revendication 4, **caractérisé en ce que** le dispositif d'amenée de liquide (2) est en forme d'entonnoir et est agencé avec une ouverture contre la membrane (3) ou contre le générateur d'aérosol à membrane (4), de telle sorte qu'un liquide de nettoyage versé dans le dispositif d'amenée de liquide (2) en forme d'entonnoir est en contact avec la membrane (3).

10. Dispositif de nettoyage selon l'une des revendications 4 à 9, **caractérisé en ce qu'**il est prévu sur le dispositif d'amenée de liquide (2) un joint d'étanchéité (11) qui empêche la sortie du liquide de nettoyage contre la membrane (3) ou contre le générateur d'aérosol à membrane (4).

11. Dispositif de nettoyage selon l'une des revendications 4 à 10, **caractérisé en ce qu'**il est prévu un récipient collecteur (10) pour collecter le liquide de nettoyage sortant du côté du liquide de la membrane.

12. Dispositif de nettoyage selon la revendication 10, **caractérisé en ce qu'**il est prévu dans le récipient collecteur (10) un logement (15) pour placer le générateur d'aérosol à membrane (4).

13. Dispositif de nettoyage selon la revendication 11, **caractérisé en ce que** le dispositif d'amenée de liquide (2) et le récipient collecteur (10) sont conçus de manière à pouvoir pivoter l'un par rapport à l'autre autour d'un axe (13).

14. Dispositif de nettoyage selon la revendication 13, **caractérisé en ce que** le dispositif d'amenée de liquide (2) est conçu d'une seule pièce avec un couvercle (12).

15. Dispositif de nettoyage selon l'une des revendications 4 à 14, **caractérisé en ce que** le dispositif d'activation d'oscillation (9) est un appareil de commande qui fournit un signal de commande de préférence électrique pour la commande du générateur d'aérosol à membrane (4).

16. Dispositif de nettoyage selon la revendication 15, **caractérisé en ce que** l'appareil de commande (9) est l'appareil de commande (101) de l'appareil d'inhalation thérapeutique (100).

17. Dispositif de nettoyage selon la revendication 15 ou 16, **caractérisé en ce que** le signal de commande du dispositif d'activation d'oscillation (9) correspond au signal de commande qui est envoyé au générateur d'aérosol à membrane (4) pendant le fonctionnement en nébuliseur.

18. Dispositif de nettoyage selon la revendication 15 ou 16, **caractérisé en ce que** le signal de commande du dispositif d'activation d'oscillation (9) se distingue du signal de commande d'un fonctionnement en nébuliseur, notamment par l'amplitude, la fréquence et les changements de fréquence, la durée et la séquence des intervalles de temps d'activation.
